# EUROPEAN PATENT SPECIFICATION

(11) **EP 1 663 138 B1**
(45) Date of publication and mention of the grant of the patent: **26.11.2008**
(21) Application number: 04782878.5
(22) Date of filing: 01.09.2004
(51) Int. Cl.: A61K 8/46, A61Q 11/00

(54) **DYE COMPOSITION AND METHOD FOR DETECTION OF DEMINERALIZED LESIONS IN TEETH**
FÄRBEZUSAMMENSETZUNG UND VERFAHREN ZUM NACHWEIS VON DEMINERALISIERTEN LÄSIONEN IN DEN ZÄHNEN
COMPOSITION COLORANTE ET METHODE DE DETECTION DE LESIONS DEMINERALISEES DANS LES DENTS

(30) Priority: 11.09.2003 US 502152 P; 20.07.2004 US 894553
(43) Date of publication of application: 07.06.2006
(73) Proprietor: THE PROCTER & GAMBLE COMPANY, Cincinnati, Ohio 45202 (US)
(72) Inventor: RAMJI, Nivedita, Mason, OH 45040 (US); FITZGERALD, Jamesina, Anne, Trenton, OH 45067 (US)
(74) Representative: Clemo, Nicholas Graham
(86) International application number: PCT/US2004/028466
(87) International publication number: WO 2005/025528

(56) References cited:
- FR-A- 2 463 613
- US-A- 3 903 252
- US-A- 5 064 640
- C. SCHÄFER: "Färbetabletten bei Zahnbelägen-was ist zu beachten?" ZAHNÄRZTLICHE NEWSLETTER, 15 June 2002 (2002-06-15), pages 1-2, XP002310336 Retrieved from the Internet: URL:www.ziis.de/newsletter_06a_02.htm> [retrieved on 2004-12-08]
- STOOKEY G K ET AL: "Dental caries diagnosis." DENTAL CLINICS OF NORTH AMERICA. OCT 1999, vol. 43, no. 4, October 1999 (1999-10), pages 665-677 , vi, XP009041364 ISSN: 0011-8532

## Description

### TECHNICAL FIELD

The present invention relates to a method for detecting demineralized lesions. The present invention also relates to compositions comprising selected blue and red or yellow dye systems, for detecting demineralized lesions such as early caries lesions (e.g. white spots), caries lesions, dental erosion, and/or orthodontically-induced demineralization.

### BACKGROUND ART

While caries rates have been on the decline across a variety of geographies in the past 40 years, worldwide caries remains a prevalent disease that is still the primary cause of tooth loss. Fluoride, delivered through community water fluoridation and fluoridated dentifrices, has been shown to be the most cost effective public health mechanism for preventing tooth decay. However, access to fluoride remains a problem worldwide. In the United States, where fluoridation of community drinking water was instituted in 1945, a significant proportion of the population, as high as 40%, still does not have access to drinking water with optimal levels of fluoride. In addition, excessive dietary intake of sugars and poor oral hygiene habits can further contribute to the development of demineralized lesions and frank caries. Thus, the worldwide incidence of caries is still significant.

The prior art teaches the use of visual tactile methods, the use of certain dyes, and the use of X-rays for the detection of caries lesions. For example, caries detection via the use of dyes is discussed in the following references: US 6,084,005, Fukunishi et al, issued July 4, 2000 (to facilitate the removal of the infection portion of the tooth during drilling and restoration); US 4,347,233, Yamauchi et aL, issued Aug. 31, 1982 (teaches the use of dyes to distinguish between the sound tooth and caries infected portion of the tooth); Dental caries diagnosis, Stookey et al., Dental Clinics of North America, Oct. 1999, vol.43 No.4, pp 665-677 (reviews the use of dyes for assisting light fluorescence for caries detection); and Caries detector dyes-An in vitro assessment of some new compounds, Ansari et al, J. of Oral Rehabilitation, 1999 26, 453-458 (dyes lack specificity as caries detectors).

Dental professionals may also use visual methods, tactile methods, and X-rays to aid in the detection of caries lesions in the dentist's office.

All of these tools, including the use of dyes, lack specificity for recognizing demineralized lesions and the early signs of demineralized lesions such as white spots, as well as lack specificity in adequately distinguishing between sound tooth versus caries-infected portions of the tooth. There is also a need for diagnostic methods that provide safer alternatives to the use of X-rays for caries detection. Thus, there remains a need not only for improved caries prevention or treatment products and treatment regimens, but also for diagnostic methods for selectively detecting demineralized lesions.

Dyes have also been used for the detection of plaque. Zahnärztliche Newsletter Nr. 06a/2002 discusses Mira-2-Ton, a commercial product from Hago & Werfeen GmbH, which uses a combination of two dyes.

The present invention provides a simple, quick, and inexpensive means for detecting the "mineralization health" of teeth or the degree of demineralization of the teeth The subject can use the present invention as a self-diagnostic tool to more frequently monitor the progress of remineralization treatments such as the use of fluoride-containing dentifrices or improved oral hygiene practices. Once demineralization is found, and after the application of remineralization agents, the subject can reapply the present invention as often as desired to monitor how much of the tooth is being remineralized after treatment. Since the present invention is simple to use and inexpensive, the subject can monitor progress more often, especially if significant demineralization is present. Moreover, detection of early caries lesions enables early treatment with remineralization agents, at a point where some reversal or remineralization of the condition may be possible. Early caries detection allows for the earlier initiation of treatment. If the degree of demineralization is not too extensive then treatment of the lesion with remineralization agents may prevent the formation of a frank caries lesion. Thus the drilling, removal of tooth structure, and filling the lesion, (e.g. restoration) may be minimized or avoided if treatment is started before the lesion becomes irreversible.

Furthermore, the present invention can guide the dental professional in deciding how much of the tooth structure to remove and restore and in avoiding removal of sound portions of the tooth, during restoration.

The present invention may be safer and less expensive since it requires only visual observation and does not require me use of lasers, other types of exaggerated light sources, or X-rays to highlight the demineralization lesions.

### SUMMARY OF THE INVENTION

The present invention relates to the use of a blue dye, together with a red dye or a yellow dye, in the manufacture of a kit for visually highlighting demineralization lesions in tooth surfaces wherein the kit comprises:
a) a first composition comprising from 0.001% to 10%, by weight of the composition of a blue dye or mixtures thereof; and
b) a second composition comprising from 0.001% to 10%, by weight of the composition of a red dye or a yellow dye, or mixtures therefor;
wherein the first and second compositions each comprise a safe and effective amount of a solvent for the dyes, wherein the dyes are soluble in the solvent and the visual highlighting comprises sequentially applying the first composition, followed by the second composition, to the tooth surface

The first dye composition and second dye composition are applied, sequentially, to a human or animal subject's oral cavity or teeth, in need thereof. After the dye compositions are applied, the teeth are visually observed to assess demineralized lesions stained by the dyes.

### BRIEF DESCRIPTION OF THE DRAWINGS

While the specification concludes with claims that particularly point out and distinctly claim the present invention, it is believed that the present invention will be understood better from the following description of embodiments, taken in conjunction with the accompanying drawings, in which like reference numerals identify identical elements. The present oral care dye compositions may be used with an integral carrier. The integral carrier includes a strip of material, dental tray, sponge material, and mixtures thereof. In one embodiment of the present invention, the integral carrier comprises a strip of material. The strip of material is attached to the teeth via the composition or via an attachment means that is part of the integral carrier, e.g. the integral carrier may optionally be of sufficient size and/or width and have sufficient adhesiveness, that, once applied, the integral carrier overlaps with the oral soft tissues rendering more of the teeth surface available for contact with the oral care dye composition.

Without intending to limit the invention, the strip of material embodiment is described in more detail below:
FIG. 1 is a perspective view of a substantially flat strip of material having rounded comers;
FIG. 2 is a perspective view of an embodiment of the present invention, disclosing the strip of FIG. 1 upon which a second layer composition comprising an oral care dye composition described herein wherein the dyes are releaseably associated with the integral carrier and/or the present dye composition;
FIG. 3 is a cross-sectional view, taken along section line 3-3 of FIG. 2, showing an example of the strip of material having a thickness less than that of the second layer coated thereon;
FIG. 4 is a cross-sectional view, showing an alternative embodiment of the present invention, showing shallow pockets in the strip of material, which act as reservoirs for additional amounts of the second layer coated on the strip;
FIG. 5 is a cross-sectional plan view, showing an alternative embodiment for applying the second layer composition to adjacent teeth having the strip of material conforming thereto and adhesively attached to the teeth by means of the second layer composition located between the teeth and the strip of material;
FIG. 6 is a cross-sectional elevation view of a tooth, taken along section line 6-6 of FIG. 5, showing the strip of material of the present invention conforming to and adhesively attached to the teeth by means of the second layer composition located between the teeth and strip of material;
FIG. 7 is a cross-sectional plan view, similar to FIG. 5, showing a strip of material of the present invention conforming to the teeth and the adjoining soft tissue and adhesively attached to both sides of the teeth by means of the second layer composition located between the teeth and the strip of material;
FIG. 8 is a cross-sectional elevation view, taken along section line 8-8 of FIG.7, showing a strip of material of the present invention conforming to both the tooth and the adjoining soft tissue and adhesively attached to both sides of the tooth by means of the second layer composition located between the teeth and the strip of material;
FIG. 9 is a perspective view of an alternative embodiment of the present invention, disclosing the strip of material coated with a second layer composition of FIG. 2 for treating teeth and adjoining soft tissue having a release liner;
FIG. 10 is a cross-sectional view of an alternative embodiment of the present invention, taken along section line 10-10 of FIG. 9, showing a release liner attached to the strip of material by the second layer composition on the strip of the material.

### DETAILED DESCRIPTION

### Definitions

By "anticalculus" or "antitartar" agent, as used herein, means a material effective in reducing, controlling, inhibiting, preventing, and/or minimizing mineral (e.g., calcium phosphate) deposition related to calculus or tartar formation.

By "demineralized lesion" as used herein, includes early caries lesions, caries lesions, frank caries lesions (clinically relevant lesion), D₁, D₂, D₃ and D₄ lesions under DMFS or DMFT scoring methods, dental erosion, orthodontically-induced demineralized lesions, in any tooth surface including smooth, occlusal tooth surfaces or roots of teeth. The term "demineralized lesions" as used herein does not include plaque and calculus.

By "early caries lesions" as used herein, means white spots, which are demineralized regions of the tooth which are reversible, e.g. the surface of the tooth may be restored with remineralizing agents or by improving oral hygiene habits of the subject.

By "caries lesions" as used herein means tooth decay which is the progressive decalcification of the enamel and dentin of a tooth characterized by a DMFS or DMFT score of D₂, D₃ and D₄.

By "dental erosion" as used herein means a permanent loss of tooth substance from the surface caused by extrinsic (oral consumption of dietary acids such as acidic beverages or fruit juices and environmental factors such as exposure to airborne contamination or acidic water in swimming pools) or intrinsic factors (endogeous acids produced in the stomach and which contact the teeth during the processes of vomiting, regurgitation or reflux), as opposed to subsurface demineralization or caries caused by bacterial action.

By "orthodontically induced demineralized lesions" as used herein means early caries lesions, caries lesions, frank caries lesions, dental erosion, or other types of teeth demineralization that is caused by the subject's wearing of orthodontic device such as mouth guards, braces, retainers, etc.

By "mineralization health of teeth" as used herein, means the level of mineral content of the teeth or the degree to which mineral is absent due to the presence and extent of demineralized lesions, or other state of demineralization.

A "visual-tactile caries assessment" is an examination performed on the oral cavity, and specifically the tooth or tooth surfaces of an individual to determine the integrity of the tooth or tooth surface. The examination comprises a visual assessment to locate white spot or open lesions and a tactile assessment with the use of dental implements or explorers to locate decayed, softened or "sticky" lesions on the teeth or tooth surfaces. Where a strict visual caries assessment is conducted, no tactile evaluation is performed. DMFS or DMFT are the typical scoring methods used in the visual-tactile caries assessments.

The term "DMFS" as used herein is indicative of the scoring method used to evaluate the tooth surface in the visual-tactile caries assessments of the human subjects. In this scoring method, each non-sound tooth surface receives a score of either "D", which indicates decay, "M", which indicates missing, or "F", which indicates filled. The "S", stands for (tooth) surface. A score of "D" may be further classified as D₁, D₂, D₃ and D₄ each indicating a different diagnostic threshold that relates to the severity of dental caries. D₁ denotes clinically detectable enamel lesions with intact surfaces. D₂ denotes clinically detectable "cavities" limited to enamel. D₃ denotes clinical detectable lesions in dentine, open or closed. D₄ denotes lesions extending into the pulp chamber. In accordance with ADA guidelines, surfaces scored as decayed must have evidence of having a clinically relevant carious lesion. Only surfaces demonstrating visible and/or tactile loss of tooth structure are considered to reflect a clinically relevant carious lesion. Thus, in one embodiment of the instant invention, only lesions characterized by D₂, D₃ or D₄ are used to identify a tooth surface as decayed. In one embodiment, surfaces that show evidence of demineralization in the absence of a tactile change denoting loss of enamel structure, D₁ are sound (including incipient lesions, white spot lesions and hypomineralized intact surfaces). In DMFS scoring one score is given for each tooth surface. Five surfaces per posterior tooth, specifically buccal, lingual, occlusal, mesial and distal, and four surfaces per anterior tooth, specifically buccal, lingual, mesial and distal, are scored. A total DMFS score is given to each subject, the number indicating the total number of tooth surfaces that are decayed, missing or filled.

The term "DMFT" as used herein is an alternate scoring method that may be employed when visual-tactile assessments are conducted of the teeth. In DMFT scoring, "D", "M" and "F" indicate decayed, missing and filled, respectively. The "T" indicates tooth. In the instant invention teeth are further categorized as D₁, D₂, D₃ and D₄ as described above. In DMFT scoring only one score is given for each whole tooth, rather than each individual tooth surface being scored independently. The total DMFT score given to each subject reflects the total number of teeth that are decayed, missing or filled.

By the term "caries prevention or treatment product" as used herein is meant any product with caries efficacy potential, for example, a product which is not intentionally swallowed for purposes of systematic administration of anti-caries agents, but is retained on tooth surfaces for a sufficient time to contact all of the dental surfaces. The product may be in any desired form. In one embodiment the caries prevention or treatment product is a dentifrice product in toothpaste form that contains a fluoride ion source.

The term "restorative dentistry" as used herein means any treatment, material or device that restores a tooth surface, or replaces a tooth or all teeth and adjacent tissue.

By "oral care dye composition" or "oral care composition" as used herein is meant a product which is not intentionally swallowed for purposes of systemic administration of agents or therapeutic agents, but is retained in the oral cavity for a sufficient time to contact some or substantially all of the hard dental surfaces. In addition this term can mean a product which may be intentionally swallowed but not swallowed for the purposes of systemic administration of agents or therapeutic agents.

By "safe and effective amount" as used herein is meant an amount of a component, high enough to significantly (positively) modify the condition to be treated or to effect the desired result, but low enough to avoid serious side effects (at a reasonable benefit/risk ratio), within the scope of sound medical/dental judgment. The safe and effective amount of a component, will vary with the particular condition (e.g., to diagnosis caries, etc.) being treated, the age and physical condition of the patient being treated, the severity of the condition, the duration of treatment, the nature of concurrent therapy, the specific form employed, and the particular vehicle from which the component is applied.

By "tooth surfaces" as used herein is meant the pits, fissures, occlusal surfaces, cleft, crevices, grooves, depressions, interstices, irregularities, inter-proximal surfaces between the teeth and/or along the gum line, the smooth surfaces of teeth, and/or the grinding or biting surfaces of a tooth.

All percentages and ratios used hereinafter are by weight of total composition, unless otherwise indicated.

All measurements referred to herein are made at 25°C unless otherwise specified.

All percentages, ratios, and levels of ingredients referred to herein are based on the actual amount of the ingredient, and do not include solvents, fillers, or other materials with which the ingredient may be combined as a commercially available product, unless otherwise indicated.

### DYES

The dyes of the present invention selectively stain demineralized lesions making these lesions visible via the "naked eye" at visible light wavelengths. The dye concentration in the present compositions generally ranges from 0.001% to 10% by weight, in another embodiment from 0.01% to 3%, by weight, in yet another embodiment from 0.1% to 1%, by weight. The first composition comprises any blue dye, and the second composition comprises any red or yellow dye.

In one embodiment, the blue dyes are selected from the group consisting of those listed in Table 1 (and mixtures thereof):

**Table 1**

| **List of Blue Colorants** | | | |
|---|---|---|---|
| **Color Index Name** | **CAS Number and CI Number** | **Other Names** | **Description/Chemical Name** |
| Acid Blue 1 | 129-17-9 | 42045 (European Name) | Hydrogen [4-[4- (diethylamino)- 2', 4'-disulphonatobenzhydrylidene]cyclohexa - 2, 5- dien- 1-ylidene]diethylammonium, sodium salt |
| Acid Blue 3 | 3536-49-0 | 42051 (European Name) | Bis[hydrogen [4-[4- (diethylamino)- 5'-hydroxy- 2', 4'-disulphonatobenzhydrylidene]cyclohexa -2, 5- dien- 1-ylidene]diethylammonium], calcium salt |
| Acid Blue 5 | 3374-30-9 | Acid Blue 5 (Japanese name Blue #202) | |
| Acid Blue 5 | 3374-30-9 | Acid Blue 5 (Japanese name Blue #203) | Benzenemethanaminium,N-ethyl-N-[4-[[4-ethyl(phenylmethyl)amino]phenyl](5-hydroxy-2,4-disulfophenyl)methylene]-2,5-cyctohexadien-1-ylidene]-,hydroxide,inner salt,calcium salt(2:1) |
| Acid Blue 7 | 3486-30-4 | 42080 (European Name) | Hydrogen (benzyl)[4- [[4-[benzylethylamino]phenyl](2,4-disulphonatophenyl)methylene]cyclohex a- 2, 5- dien- 1-ylidene](ethyl)ammonium, sodium salt |
| Food Blue 2 | 2650-18-2 | 42090 (European Name) Blue # 1 (Japan) FD & C Blue # 1 (US) | Diammonio(ethyl)[4- [[4- [ethyl(3-sulphonatobenzyl)amino]phenyl](2-sulphonatophenyl)methylene]cyclohexa-2, 5- dien- 1- ylidene](3-sulphonatobenzyl)ammonium |
| Acid Blue 9 | 3844-45-9 | 42090 (European Name) Blue # 205 (Japan) FD&C Blue # 4 | Dihydrogen (ethyl)[4- [4-[ethyl(3-sulphonatobenzyl)]amino]- 2'-sulphonatobenzhydrylidene]cyclohexa-2, 5- dien- 1- ylidene](3-sulphonatobenzyl)ammonium, disodium salt |
| Basic Blue 26, Pigment Blue 2 | 2580-56-5 | 44045 (Europe) | [4- [[4- anilino- 1- naphthyl][4-(dimethylamino)phenyl]methylene]cycl ohexa- 2, 5- dien- 1-ylidene]dimethylammonium chloride |
| Solvent Blue 63 | 6408-50-0 | Blue # 403 (Japan) | |
| Acid Blue 80 | 4474-24-2 | 61585 (Europe) | Sodium 3, 3'- (9, 10- dioxoanthracene- |
| | | | 1, 4- diyldiimino)bis(2, 4, 6-trimethylbenzenesulphonate |
| Acid Blue 62 | 4368-56-3 | 62045 (Europe) | Sodium 1- amino- 4-(cyclohexylamino)- 9, 10- dihydro- 9, 10- dioxoanthracene- 2- sulphonate |
| Vat Blue 4 | 81-77-6 | 69800 (Europe) | 6, 15- dihydroanthrazine- 5, 9, 14, 18-tetrone |
| Vat Blue 6; Pigment Blue 64 | 130.20-1 | 69825 (Europe) Blue #204 (Japan) | 7,16- dichloro- 6, 15-dihydroanthrazine- 5,9,14,18- tetrone |
| Vat Blue 1; Pigment Blue 66 | 482-89-3 | 7300 (Europe) Blue #201 (Japan) | 2- (1, 3- dihydro- 3- oxo- 2H- indazol-2- ylidene)- 1,2- dihydro- 3H- indol- 3-one |
| Acid Blue 74; Food Blue 1 | 860-22-0 | 73015 (Europe) Blue #2 (Japan) | Disodium 5, 5'- (2- (1, 3- dihydro- 3-oxo- 2H- indazol- 2- ylidene)- 1,2-dihydro- 3H- indol- 3- one)disulphonate |
| Pigment Blue 16 | 574-93-6 | 74100 (Europe) | 29H, 31H- phthalocyanine |
| Pigment Blue 15 | 147-14-8 | 74160 (Europe) Blue #404 (Japan) | 29H, 31H- phthalocyaninato(2- )- N29, N30, N31, N32 copper |
| Direct Blue 86 | 1330-38-7 | 74180 (Europe) | Disodium [29H, 31H-phthalocyaninedisulphonato(4- )- N29, N30, N31, N32]cuprate(2- ) |
| Pigment Blue 29 | 1302-83-6 | 77007 (Europe) Lazurite Ultramarine (Japan) Ultramarines (US) | |
| Pigment Blue 27 | 14038-43-8 | Prussian blue; Ferric FerroCyanide (Japan, US) 77510 (Europe) | |
| Pigment Blue 27 CI 77520 | 14038-43-8 | Prussian blue; Ferric Ammonium Ferrocyanide (Japan, US) | |
| Bromothymol Blue | 76-59-5 | Bromothymol Blue (Europe) | 3' 3" dibromothymolfonpthalein C₂₇H₂₈Br₂O₅₃ |
| Guaiazulene Blue | 489-84-9 | Guaiazulene (Japan, US) | Isopropyl-7-dimethyl-4,4-azulene |

In another embodiment the blue dye is selected from the group consisting of Acid Blue 9, Food Blue 1, Food Blue 2, and mixtures thereof.

In one embodiment, the red or yellow dye is selected from the group consisting of those listed in Table 2 (including salts, sodium salts, potassium salts, free acids, free bases, lakes thereof, and mixtures thereof)¹:

**TABLE 2**

| **Color Index Name** | **CAS Number and CI Number** | **Other Names'** | **Description/Chemical Name** |
|---|---|---|---|
| Acid Yellow 73 | 518-47-8 | Yellow No. 11 (EC-former) | Fluorescein sodium salt Hydroxy-Phthaleins |
| | CINo. 45350 | Yellow No. 202(1) (Japan) D&C Yellow No. 8 (US) Uranine Na Salt (Other) | |
| Acid Yellow 73 | 6417-85-2 | Yellow No. 202(2) (Japan) | Hydroxy-Phthaleins |
| | CI 45350 | Uranine K Salt (Other) | Fluorescein potassium salt |
| Acid Yellow 73 | 2321-07-5 | Yellow No. 201 (Japan) | Hydroxy-Phthaleins |
| | CI 45350:1 | D&C Yellow No. 7 (US) Fluorescein Free Acid (Other) | Fluorescein free acid |
| Solvent Red 72 | 596-03-2 CI 45370:1 | Red No. 17 (EC - former) Orange No. 201 (Japan) D&C Orange No. 5 (US) Dibromo Fluorescein Free | Hydroxy-Phthaleins 4,5-Dibrom-fluorescin, free acid |
| | | Acid (Other) | 4,5-Dibrom-fluorescin, sodium salt (CI 45370) |
| Acid Red 87 | 17372-87-1 CI 45380 | Red No. 18 (EC - former) Red No. 230(1) (Japan) D&C Red No. 22 (US) Eosine Na Salt (Other) | Hydroxy-Phthaleins (sodium salt) |
| Acid Red 87 | 548-26-5 CI 45380 | Red No. 230(2) (Japan) Eosine K Salt (Other) | Hydroxy-Phthaleins (potassium salt) |
| Pigment Red 90 | 1326-05-2 CI 45380:1 | | |
| Solvent Red 43 | 15086-94-9 CI 45380:2 or :1 | Red No. 18 (EC - former) Red No. 223 (Japan) D&C Red No. 21 (US) | Hydroxy-Phthaleins (free acid) |
| | | Tetrabromo Fluorescein Free Acid | |
| Acid Red 98 | 6441-77-6 CI45405 | Phloxine Na Salt (Other) | 2,4,5,7-Tetrabrom-3"6"-diclorofluorescin, potassium salt |
| Acid Red 92 | 18472-87-2 CI 45410 | Red No. 20 (EC -former) Red No. 104(1) (Japan) D&C Red No. 28 (US) Phloxine B Na Salt (Other) | Hydroxy-Phthaleins 2,4,5,6-tetrabromo-3",4",5",6"-tetrachloro-fluorescein, sodium salt |
| Acid Red 92 | 13473-26-2 | Red No. 231 (Japan) | Hydroxy-Phthaleins |
| | CI 45410 | Phloxine B K Salt (Other) | Potassium salt |
| Solvent Red 48 | 13473-26-2 CI 45410:1 . | Red No. 218 (Japan) D&C Red No. 27 (US) Phloxine B Free Acid (Other) | Hydroxy-Phthaleins 2,4,5,6-tetrabromo-3",4",5",6"-tetrachlorofluorescein free acid |
| Acid Red 92/ Pigment Red 174 | 15876-58-1 CI 45410:2 | | |
| Acid Red 95 | 33239-19-9 CI 45425 | Red No. 21 (EC-former) Orange No. 207 (Japan) | 2,4-Diiodide-fluorescin, sodium salt |
| | | D&C Orange No. 11 (US) Diiodo Fluorescein Na Salt (Other) | Hydroxy-Phthaleins |
| Solvent Red 73 | 38577-97-8 CI 45425:1 | Orange No. 206 (Japan) D&C Orange No. 10 (US) | 2,4-Diiodide-fluorescin, free acid |
| | | Diiodo Fluorescein Free Acid (Other) | Hydroxy-Phthaleins 2,4-Diiodide-fluorescin, aluminum lake (CI 45425:2) |
| Acid Red 51/ Food Red 14 | 16423-68-0 CI 45430 | Red No. 22 (EC - former) Red No. 3 (Japan) FD&C Red No. 3 (US) | 2,4,5,7-Tetraiodide-fluorescin, sodium salt |
| | | Erythrosine Na Salt (Other) | Hydroxy-Phthaleins |
| Pigment Red 172 | 12227-78-0 CI 45430:1 | | 2,4,5,7-Tetraiodide-fluorescin, aluminum lake 2,4,5,7-Tetraiodide-fluorescin, free acid (CI 45430:2 -Solvent Red 140) |
| Acid Red 94 | 632-68-8 CI 45440 (Other) | Red No. 232 (Japan) Rose Bengal K Salt (Other) | Hydroxy-Phthaleins |
| Acid Red 94 | 632-69-90 CI 45440 | Red No.105(1) (Japan) Rose Bengal Na Salt (Other) | 2,4,5,7-tetraiodide-3',4',5',6' -tetrochloro fluorescein sodium salt or potassium salt |
| Solvent Orange 16 | 24545-86-6² CI 45396 | | Xanthene dye 4,5-Dinitro-fluorescin, free acid Hydroxy-Phthaleins |
| C.I. Acid Red 52 | 3520-42-1 CI 45100 | Red No. 102 (Japan) Sulforhodamin B | Xanthene dye 3,6-Bis(diethylamino)-9-(2,4-disulfophenyl)-xanthylimmonium, sodium salt |
| C.I. Acid Red 50 | 5873-16-5 CI 45220 | Sulforhodamin G | Xanthene 3,6-Bis(diethylamino)-2,7-dimethyl-9-(2,4-disulfophenyl)-xanthylimmonium, sodium salt |
| Acid Red 289 | 12220-28-9 | | |
| Basic Red 1 | 989-38-8 CI 45160 | Rhodamine 6G | |
| Pigment Red 81 | 12224-98-5 CI 45160:1 | | |
| Solvent Red 49 | 509-34-2 CI 45170:1 | Rhodamine B Base | |
| Basic Violet 11 | 2390-63-8 CI 45175 | Fanal Red 6BM (IG) | |
| Mordant Red 27 | 6539-22-4 CI 45180 | Chromoxane Brilliant Red | |

| | | | |
|---|---|---|---|
| ¹ See the CTFA International Color Handbook, 2nd Ed., The Cosmetic, Toiletry, and Fragrance Association, Inc., 1985, 1992, Including pp. 185-201. Also see, Coloring of Food, Drugs, and Cosmetics, G. Otterstatter, Marcel Dekker, Inc. 1999, including Chapter 9 pp. 222-236. ² Sigma Aldrich Product No. 35,882-7: C2OH10N2O9. | | | |

In another embodiment the red or yellow dye is C.I. Acid Red 52.

### SOLVENT

The compositions of the present invention further comprise a safe and effective amount of a solvent. The solvent is present at a level of from 30% to 99.999%, in another embodiment from 60% to 98% and in yet another embodiment from 70% to 95% by weight of the composition.

The solvent for the composition of the invention must be selected so that the dye is soluble in the solvent The solvent is also selected to provide a viscosity of the composition to facilitate the penetration of the composition into the infected dentin. Solvents include water-miscible solvents which are easily miscible with water at any ratio rendering a homogeneous solutions.

In one embodiment the solvent is selected from the group consisting of organic mono-, di- or tri-hydroxy compounds having from 2 to 10 carbon atoms. Examples of suitable monohydroxy compounds include monohydric alcohols such as ethanol, n-propanol, isopropanol, isobutyl alcohol, n-amyl alcohol, isoamyl alcohol, neopentyl alcohol, 2-hexanol, 1-heptanol, nonyl alcohol and decyl alcohol. Solvents also include monoethers of dihydric alcohols such as ethylene glycol monomethyl ether, ethylene glycol monoethyl ether, ethyleneglycol monobutyl ether, diethylene glycol monoethyl ether, diethylene glycol monobutyl ether, triethylene glycol monomethyl ether, triethylene glycol monoethyl ether, tetraethylene glycol monomethyl ether and tetraethylene glycol monoethyl ether. Solvents also include monoesters of dihydric alcohols such as ethylene glycol monoacetate and diethylene glycol monoacetate. Examples of suitable dihydroxy compounds include dihydric alcohols such as ethylene glycol, propylene glycol, 1,3-propanediol, 1,2-butanediol, 1,4-butanediol, 1,2-propylene glycol, 1,3-propylene glycol, 1,3-butanediol, diethylene glycol, triethylene glycol, tetraethylene glycol, pentaethylene glycol and dipropylene glycol, and monoethers and monoesters of trihydric alcohols such as glycerin monomethyl ether and glycerin monoacetate, ethylene glycol monoacetate, diethylene glycol monoacetate. Examples of suitable trihydroxy compounds include glycerol and pentaglycerol. Other solvents include tetrahydrofuran, dimethylformamide, dimethylsulfoxide, dioxane, acetone and dimethoxyethane, and the like. Mixtures of the above solvents can be used. In another embodiment the solvent is selected from the group consisting of 1,3-propylene glycol, propylene glycol, 1,2 propylene glycol, dipropylene glycol, and mixtures thereof. In another embodiment the solvent is propylene glycol.

### Combination of Integral Carrier and Dye Composition

In one embodiment the present invention relates to a delivery system comprising the present oral care dye composition used with an integral carrier. In one embodiment the integral carrier is selected from the group consisting of a strip of material, dental tray, a sponge material and mixtures thereof. In one embodiment the delivery system comprises: a first layer of a strip material; a second layer comprising the above oral care dye compositions, whereby the dye is releasably associated with the composition and/or the strip material.

### I. First Layer

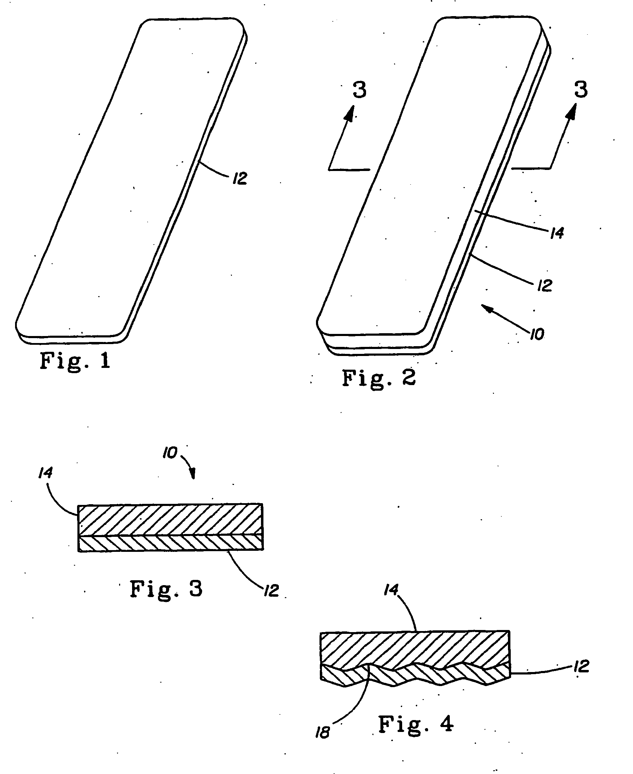

Referring now to the drawings, and more particularly to FIGS. 1 and 2, there is shown a first embodiment of the present invention, generally indicated as 10, representing a delivery system for delivering dye to the teeth. Delivery system 10 has a strip of material 12, which is substantially flat, preferably with rounded corners.

Releasably applied onto said strip of material 12 is a second layer composition 14. Second layer composition 14 is, in one embodiment, a homogenous, and may be uniformly and continuously coated onto strip of material 12, as shown in FIG. 3. However, second layer composition 14 may alternatively be a laminate or separated layers of compositions, (wherein one layer comprises the first dye composition and a second layer comprises the second dye composition) separate stripes or spots or other patterns comprising the first and/or second dye compositions, or a combination of these structures including a continuous coating of second layer composition 14 along a longitudinal axis of a portion of strip of material 12.

As shown in FIG. 4 in an alternative embodiment, strip of material 12 may have shallow pockets 18 formed therein. When second layer composition 14 is coated on a strip of material 12, additional second layer composition 14, if present, fills shallow pockets 18 to provide reservoirs of second layer composition 14.

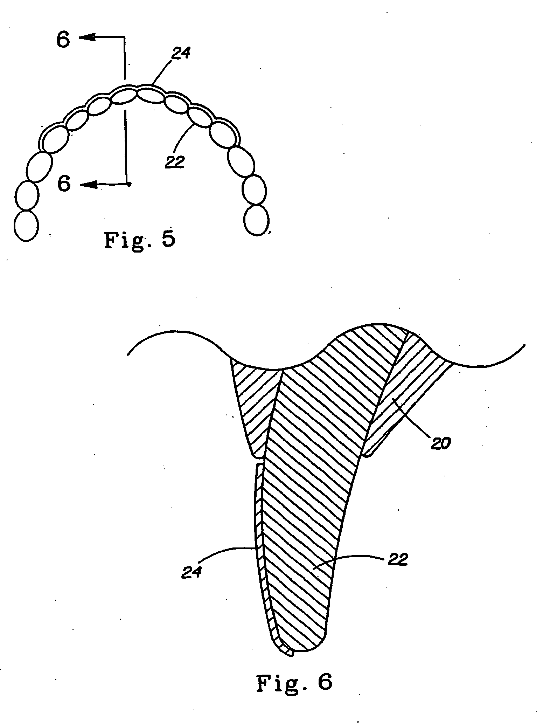

FIGS. 5 and 6 show a delivery system 24 of the present invention applied to the surface of a tooth and plurality of adjacent teeth. Embedded in adjacent soft tissue 20 are a plurality of adjacent teeth 22. Adjacent soft tissue herein defined as soft tissue surfaces surrounding the tooth structure including: papilla, marginal gingival, gingival sulculus, inter dental gingival, and gingival gum structure on lingual and buccal surfaces up to and including muco-gingival junction on the pallet.

In both FIGS. 5 and 6, delivery system 24 represents strip of material 12 and second layer composition 14, with second layer composition 14 on the side of strip material 12 facing tooth 22. Second layer composition 14 may be pre-applied to strip of material 12, or may be applied to strip of material 12 by the user prior to application to the teeth. In an alternate embodiment, the second layer composition may be applied directly to teeth 22 by the user and then covered by a strip of material 12. In any case, strip of material 12 has a thickness and flexural stiffness such that it can conform to the contoured surfaces of tooth 22 and to adjacent soft tissue 20. In one embodiment, the strip of material has sufficient flexibility to form to the contours of the oral surface, the surface being a plurality of adjacent teeth. The strip of material is also readily conformable to tooth surfaces and to the interstitial tooth spaces without permanent deformation when the delivery system is applied. The delivery system can be applied without significant pressure.

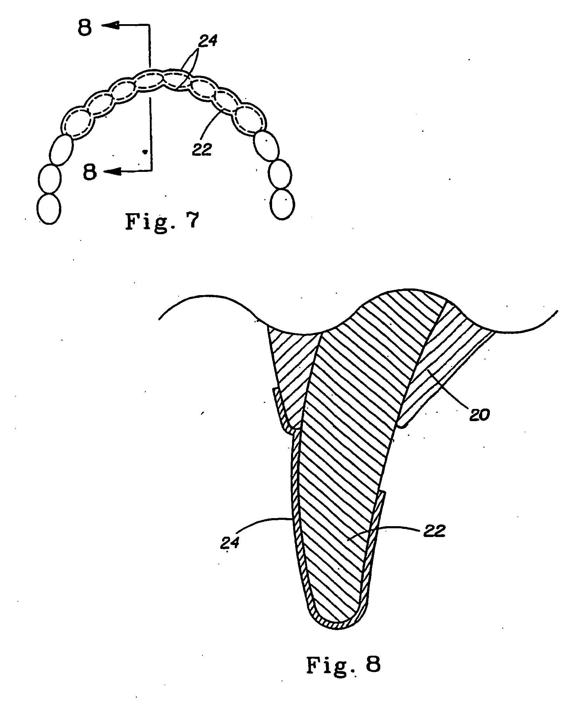

FIGS. 7 and 8 show a delivery system 24 of the present invention applied to both front and rear surfaces of a plurality of adjacent teeth 22 as well as to adjacent soft tissue 20. Delivery system 24 represents strip of material 12 and second layer composition 14, with second layer composition 14 on the side of strip of material 12 facing tooth 22.

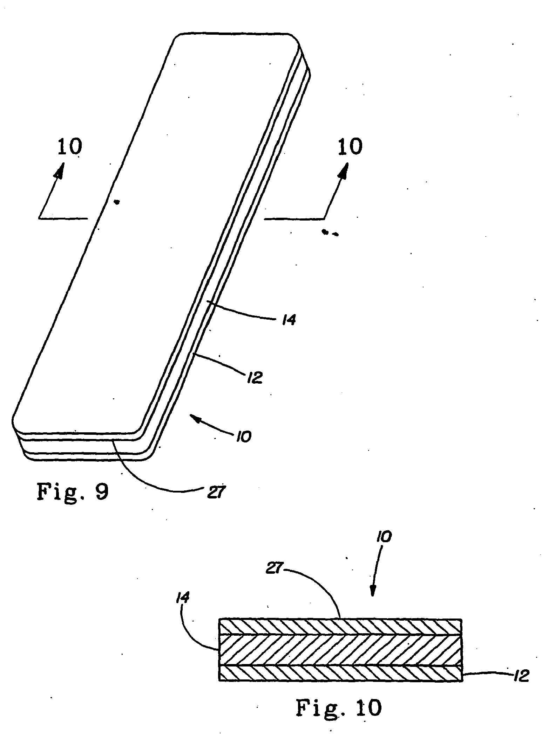

FIGS. 9 and 10 show an optional release liner 27. Release liner 27 is attached to strip of material 12 by second layer composition 14. Second layer composition 14 is on the side of strip material 12 facing release liner 27. This side is applied to the tooth and gum surfaces once release liner 27 is removed.

In one embodiment the first layer of the delivery system of the present invention is comprised of a strip of material. Such first layer materials are described in more detail in U.S. Pat. Nos; 6,136,297; 6,096,328; 5,894,017; 5,891,453; and 5,879,691, all to Sagel, et al., and all assigned to The Procter & Gamble Company, and in U.S. Pat. Nos. 5,989,569 and 6,045,811 both to Dirksing, et al., and both assigned to The Procter & Gamble Company.

The strip serves as a protective barrier for the dye. It prevents leaching and/or erosion of the second layer by for example, the wearer's tongue, lips, and saliva. This allows the second layer composition to act upon the hard surfaces of the oral cavity for the correct exposure time periods, described herein.

The strip material may comprise polymers, natural and synthetic woven materials, nonwoven material, foil, paper, rubber, foam, and combinations thereof. The strip material may be a single layer of material or a laminate of more than one layer. Regardless of the number of layers, the strip of material is substantially water insoluble. The strip may also be water impermeable. In one embodiment the material is any type of polymer or combination of polymers that meet the required flexural rigidity and are compatible with the compositions. Suitable polymers include, but are not limited to, polyethylene, ethylvinylacetate, polyesters, ethylvinyl alcohol and combinations thereof. Examples of polyesters include Mylar® and fluoroplastics such as Teflon®, both manufactured by Dupont. In one embodiment the material is polyethylene. The strip of material is generally less than about 1 mm (millimeter) thick, in one embodiment less than about 0.05 mm thick, in yet another embodiment from about 0.001 to about 0.03 mm thick. A polyethylene strip of material is generally less than about 0.1 mm thick and in one embodiment from about 0.005 to about 0.02 mm thick.

In one embodiment the shape of the strip of material is any shape and size that covers the desired oral hard tissue surface. In one embodiment the strip has rounded corners to avoid irritation of the soft tissue of the oral cavity. "Rounded corners," means not having any sharp angles or points. In one embodiment, the length of the strip material is such that it will cover all of the upper or lower teeth, in another embodiment from about 2 cm (centimeter) to about 12 cm, in another embodiment from about 4 cm to about 9 cm. The width of the strip material will also depend on the number of teeth area to be covered. The width of the strip is generally from about 0.5 cm to about 4 cm, in one embodiment from about 1 cm to about 2 cm. In yet another embodiment, the strip may be worn as a patch on one or several teeth to monitor a localized condition. In one embodiment the second layer composition is applied to the integral carrier in a way to avoid contact with the oral soft tissues.

The strip material may contain shallow pockets. When the composition is coated on a strip of material, the compositions or additional cosmetic and therapeutic oral care actives fill shallow pockets to provide reservoirs of these components. Additionally the shallow pockets help to provide texture to the delivery system. In one embodiment the strip material will have an array of shallow pockets. Generally the shallow pockets are approximately 0.4 mm across and about 0.1 mm deep. When shallow pockets are included in the strip of material and compositions are applied to it in various thicknesses, the overall thickness of the delivery system is less than about 1 mm. In one embodiment the overall thickness is less than about 0.5 mm.

Flexural stiffness is a material property that is a function of a combination of strip thickness, width and material modulus of elasticity. This test is a method for measuring the rigidity of polyolefin film and sheeting. It determines the resistance to flexure of a sample by using a strain gauge affixed to the end of a horizontal beam. The opposite end of the beam presses across a strip of the sample to force a portion of the strip into a vertical groove in a horizontal platform upon which the sample rests. A microammeter wired to the strain gauge is calibrated in terms of deflection force. The rigidity of the sample is read directly from the microammeter and expressed as grams per centimeter of the sample strip width. In the present invention, the strip of material has a flexural stiffness of less than about 5 grams/cm as measured on a Handle-O-Meter, model #211-300, available from Thwing-Albert Instrument Company of Philadelphia, PA as per test method ASTM D2923-95. In one embodiment the strip has a flexural stiffness less than about 3 grams/cm, in another embodiment less than about 2 grams/cm and in yet another embodiment from about 0.1 to about 1 grams/cm. Generally, the flexural stiffness of the strip of material is substantially constant and does not change during normal use. For example, the strip of material does not need to be hydrated for the strip to achieve the low flexural stiffness in the above-specified ranges.

This relatively low stiffness enables the strip of material to cover the contours of the oral surface with very little force being exerted. That is, conformity to the contours of the oral surface of the wearer's mouth is maintained because there is little residual force within the strip of material to cause it to return to its shape just prior to its application to the oral surface, i.e. substantially flat. The strip of material's flexibility enables it to contact hard or soft tissue without irritation. The strip of material does not require continuous pressure for retention against the oral surface.

In one embodiment the strip of material is held in place on the oral surface by adhesive attachment provided by the present compositions. In one embodiment the viscosity and general tackiness of the present composition to dry surfaces cause the strip to be adhesively attached to the oral surface without substantial slippage from the frictional forces created by the lips, teeth, tongue, and other oral surfaces rubbing against the strip of material while talking drinking, etc. However, this adhesion to the oral surface is low enough to allow the strip of material to be easily removed by the wearer by simply peeling off the strip of material using one's finger. The delivery system is easily removable from the oral surfaces without the use of an instrument, a chemical solvent or agent or excess friction.

In another embodiment the strip of material is held in place on the oral surface by adhesive attachment provided by the integral carrier itself. In one embodiment the strip of material can extend, attach, and adhere to the oral soft tissue. Alternatively, an adhesive can be applied to that portion of the strip of material that will attach the delivery systems to the oral soft tissue. In one embodiment the second layer composition does not contact oral soft tissues.

### Second Layer

In one embodiment the second layer comprises a safe and effective amount of the oral care dye compositions described herein.

### Optional Release Liner

In one embodiment the release liner may be formed from any material which exhibits less affinity for the second layer composition than the second layer composition exhibits for itself and for the first layer strip of material. The release liner may comprise a rigid sheet of material such as polyethylene, paper, polyester, or other material, which is then coated with a nonstick type material. The release liner may be cut to substantially the same size and shape as the strip of material or the release liner may be cut larger than the strip of material to provide a readily accessible means for separating the material from the strip. The release liner may be formed from a brittle material that cracks when the strip is flexed or from multiple pieces of material or a scored piece of material. Alternatively, the release liner may be in two overlapping pieces such as a typical adhesive bandage design. A description of materials suitable as release agents is found in Kirk-Othmer, Encyclopedia of Chemical Technology, Fourth Edition, Volume 21, pp. 207-218.

### Combination of Soft/Rigid Dental

### Trays or Sponge Material (Foams) and Dye Composition

The oral care dye compositions may be used in combination with a dental tray, for example the type of dental trays that are well known in the whitening art. The general process for preparing dental trays is known in the art. For example, an alginate impression which registers all teeth surfaces plus gingival margin is made and a stone cast is promptly made of the impression. If reservoirs are desired they are prepared by building a layer of rigid material on the stone cast on specific teeth surfaces to be treated. A dental tray is then vacuum formed from the modified cast using conventional techniques. Once formed, the tray is preferably trimmed barely shy of the gingival margin on both buccal and lingual surfaces. Enough tray material should be left to assure that all of the tooth will be covered to within about ¼ to about 1/3 mm of the gingival border upon finishing and beveling the tray periphery. In one embodiment one can scallop up and around interdental papilla so that the finished tray does not cover them. All tray edges are preferably smoothed so that the lip and tongue will not feel an edge prominence. The resulting tray, in one embodiment, provides a perfect fit of the patient's teeth optionally with reservoirs or spaces located where the rigid material was placed on the stone cast Dental trays may comprise soft transparent vinyl material having a preformed thickness from about 1 mm (0.04 inch) to about 1.5 mm (0.06 inch). Soft material is more comfortable for the patient to wear. Harder material (or thicker plastic) may also be used to construct the tray.

Other trays or foams useful herein include a rigid appliance which is fitted precisely to the patient's dental arches. A second type of rigid custom dental appliance is an "oversized" rigid custom dental appliance. The fabrication of rigid, custom dental appliances entails fabricating stone models of the patient's dental arch impressions, and heating and vacuum-forming a thermoplastic sheet to correspond to the stone models of a patient's dental arches. Thermoplastic films are sold in rigid or semi rigid sheets, and are available in various sizes and thickness. The dental laboratory fabrication technique for the oversized rigid dental appliance involves augmenting the facial surfaces of the teeth on the stone models with materials such as die spacer or light cured acrylics. Next, thermoplastic sheeting is heated and subsequently vacuum formed around the augmented stone models of the dental arch. The net effect of this method results in an "oversized" rigid custom dental appliance.

A third type of rigid custom dental appliance, is a rigid bilaminated custom dental appliance fabricated from laminations of materials, ranging from soft porous foams to rigid, non-porous films. The non-porous, rigid thermoplastic shells of these bilaminated dental appliances encase and support an internal layer of soft porous foam.

A fourth type of dental tray replaces rigid custom dental appliances with disposable U-shaped soft foam trays, which may be individually packaged, and which may be saturated with a pre-measured quantity of the composition of the present invention. The soft foam material is generally an open celled plastic material. Such a device is commercially available from Cadco Dental Products in Oxnard, Calif. under the tradename VitalWhite™. In one embodiment these soft foam trays comprise a backing material (e.g. a closed cell plastic backing material) to minimize the elution of the dye from the device, into the oral cavity to minimize staining of soft tissue, ingestion by the patient and/or irritation of the oral cavity tissues. In another embodiment the soft foam tray is encased by a nonporous flexible polymer. In another embodiment the open cell foam is attached to the frontal inner wall of the dental appliance and/or the open cell foam is attached to the rear inner wall of the dental appliance.

In one embodiment the present oral care dye compositions must be thick enough not to simply run out between the open cell structure of the foam and must be thin enough to readily pass through the open cell foam. In other words, the open cell foam material has an internal structural spacing sized relative to the viscosity of the compositions to absorb and allow the composition to pass through it.

An example of a closed cell material is a closed-celled polyolefm foam sold by the Voltek division of Sekisui America Corporation of Lawrence, Mass, under the tradename Volora which is from 1/32" to 1/8" in thickness. A closed cell material may also comprise of a flexible polymeric material.

An example of an opened cell material is an open celled polyethylene foam sold by the Sentinel Foam Products division of Packaging Industries Group, Inc. of Hyannis, Mass. under the tradename Opcell which is from 1/16" to 3/8" in thickness. Other open cell foam useful herein include hydrophilic open foam materials such as hydrogel polymers (e.g Medicell^{™} foam available from Hydromer, Inc. Branchburg, J.J.). Open cell foam may also be hydrophilic open foam material imbibed with agents to impart high absorption of fluids, such as polyurethane or polyvinylpyrrolidone chemically imbibed with various agents.

Appliances of the above type are further described in U.S. Patent Nos. 5,980,249, M.G. Fontenot, and U.S. 5,575,654, M.G. Fontenot.

The above dental appliances may be designed to be disposable or reuseable. Further dental trays usable herein are disclosed in U.S. Patent 6,368,576, Steven D. Jensen, issued April 9, 2002; U.S. Patent 6,309,625 Jensen, et al., issued Oct 30, 2001; U.S. Patent 6,183,251, Dan E. Fischer, issued February 6, 2001; U.S. Patent 6,036,943, Dan E. Fischer, issued March 14,2000; U.S. Patent 5,985,249, Dan E. Fischer, issued November 16, 1999; U.S. Patent 5,846,058, Dan E. Fischer, issued Dec 8, 1998; U.S. Patent 6,382,979, Sherrill F. Lindquist, issued May 7, 2002; U.S. Patent 5,098,303, Fischer, issued March 24, 1992, and U.S. Patent 5,855,870, Dan E. Fischer, issued January 5, 1999.

### OPTIONAL INGREDIENTS

### Pellicle Disruption Agent

The present compositions and methods herein can optionally comprise a safe and effective amount of a pellicle (or biofilm) disruption agent. As used herein the term "pellicle (or biofilm) disruption agent" means an agent effective in removing or minimizing pellicles or biofilm on teeth formed from proteins, lipids and glycolipids from the saliva and gingival crevicular fluid. Pellicle disruption agents are in one embodiment, included in the dye compositions herein at a safe and effective amount, in another embodiment at a level of from about 0.01 to about 20 %, in another embodiment from about 0.1% to about 10% by weight of the composition. Such agents may include, but are not limited to anticalculus agent described herein, enzymes such as proteases, elastases, halogenated furanones, etc.

Pellicle can also be removed by physical removal through sonication, pumicing, electric toothbrushing, brushing with abrasive toothpastes, etc.

### Thickening Agents

In one embodiment the present compositions comprise a gel. In preparing gels herein, it may generally be necessary to add some thickening material to provide a desirable consistency of the composition, to provide desirable release characteristics upon use, to provide shelf stability, and to provide stability of the composition, etc. In one embodiment thickening agents are selected from the group comprising carboxyvinyl polymers, carrageenan, hydroxyethyl cellulose, laponite and water soluble salts of cellulose ethers such as sodium carboxymethylcellulose, hydroxy propyl methyl cellulose and sodium carboxymethyl hydroxyethyl cellulose. Natural gums such as gum karaya, xanthan gum, gum arabic, and gum tragacanth can also be used. Colloidal magnesium aluminum silicate or finely divided silica can be used as part of the thickening agent to further improve texture.

Thickening agents can include polymeric polyether compounds, e.g., polyethylene or polypropylene oxide (M.W. 300 to 1,000,000), capped with alkyl or acyl groups containing 1 to about 18 carbon atoms.

In one embodiment the thickening or gelling agents includes a class of homopolymers of acrylic acid crosslinked with an alkyl ether of pentaerythritol or an alkyl ether of sucrose, or carbomers. Carbomers are commercially available from B.F. Goodrich as the Carbopol® series. Particularly preferred carbopols include Carbopol 934, 940, 941, 956, and mixtures thereof.

Thickening agents, in an amount from about 0.1% to about 15%, in another embodiment from about 0.2% to about 6%, in yet another embodiment from about 0.4% to about 5%, by weight of the total composition, can be used herein.

### Buffers

The present invention optionally includes a buffer at a level of from about 0.01 % to about 10%, and in another embodiment from about 0.1 to about 3%, in another embodiment from about 0.3 to about 2.5%, by weight of the composition, of buffer. Buffering agents, as used herein, refer to agents that can be used to adjust the pH of the compositions to a range of about pH 6.5 to about pH 10. These agents include alkali metal hydroxides, carbonates, sesquicarbonates, borates, silicates, phosphates, imidazole, and mixtures thereof. Specific buffering agents include trisodium phosphate, disodium phosphate, disodium hydrogen phosphate, sodium dihydrogen phosphate, monosodium phosphate, sodium hydroxide, potassium hydroxide, alkali metal carbonate salts, sodium carbonate, imidazole, pyrophosphate salts, citric acid, and sodium citrate, and mixtures thereof.

### Flavoring Agents

Flavoring agents may also be added to the compositions. Flavors enhance taste and aesthetics of the present compositions, since some of the added dyes cause objectionable taste. In addition flavors may also enhance the penetration of the dye into the demineralized lesions on teeth. Suitable flavoring agents include oil of wintergreen, oil of peppermint, oil of spearmint, clove bud oil, menthol, anethole, methyl salicylate, eucalyptol, 1-menthyl acetate, sage, eugenol, parsley oil, oxanone, alpha-irisone, marjoram, lemon, orange, propenyl guaethol, cinnamon, vanillin, thymol, linalool, cinnamaldehyde glycerol acetal known as CGA, and mixtures thereof. In one embodiment the flavor is selected from the group consisting of oil of spearmint, cinnamon, eugenol, peppermint, and mixtures thereof. Flavoring agents are generally used in the compositions at levels of from about 0.001% to about 20%, by weight of the composition, in another embodiment from about 0.1 to about 3%, and in yet another embodiment from about 0.8 to about 2% by weight.

### Oral Care Active Agent

The present invention may comprise a safe and effective amount of an oral care active agent selected from the group consisting of anticalculus agent, fluoride ion source, antimicrobial agents, dentinal desensitizing agents, anesthethic agents, antifungal agents, anti-inflammatory agents, selective H-2 antagonists, anticaries agents, and mixtures thereof.

The oral care active agents can be present in the present compositions and methods in suitable amounts. These levels will be known by those skilled in the art and are disclosed below.

### Anticaries Agents and Fluoride Ion Source

The present composition may comprise a safe and effective amount of an anticaries agent. In one embodiment the anticaries agent is selected from the group consisting of xylitol, fluoride ion source, and mixtures thereof. The fluoride ion source provides free fluoride ions during use of the composition. In one embodiment the oral care active agent is a fluoride ion source selected from the group consisting of sodium fluoride, stannous fluoride, indium fluoride, organic fluorides such as amine fluorides, and sodium monofluorophosphate. Sodium fluoride is the fluoride ion in another embodiment. Norris et al., US Patent 2,946,725, issued July 26, 1960, and Widder et al., US Patent 3,678,154 issued July 18, 1972, disclose such fluoride salts as well as others that can be used as the fluoride ion source. The present composition may comprise from about 50 ppm to about 3500 ppm, in another embodiment from about 100 ppm to about 3000 ppm, and in another embodiment from about 200 ppm to about 2,800 ppm, and in another embodiment from about 500 ppm to about 1,500 ppm, of free fluoride ions.

### Anticalculus Agents

The present compositions may comprise a safe and effective amount of at least one anticalculus agent. This amount is generally from about 0.01% to about 40% by weight of the composition, in another embodiment is from about 0.1% to about 25%, and in yet another embodiment is from about 4.5% to about 20%, and in yet another embodiment is from about 5% to about 15%, by weight of the composition. The anticalculus agent should also be essentially compatible with the other components of the composition.

The anticalculus agent is selected from the group consisting of polyphosphates and salts thereof; polyamino propane sulfonic acid (AMPS) and salts thereof; polyolefin sulfonates and salts thereof; polyvinyl phosphates and salts thereof; polyolefin phosphates and salts thereof; diphosphonates and salts thereof; phosphonoalkane carboxylic acid and salts thereof; polyphosphonates and salts thereof; pyrophosphates and salts thereof; polyvinyl phosphonates and salts thereof; polyolefin phosphonates and salts thereof; polypeptides; and mixtures thereof. In one embodiment, the salts are alkali metal salts. In another embodiment the anticalculus agent is selected from the group consisting of polyphosphates and salts thereof; diphosphonates and salts thereof; and mixtures thereof. In another embodiment the anticalculus agent is selected from the group consisting of pyrophosphate, polyphosphate, and mixtures thereof.

### Polyphosphates

In one embodiment of the present invention, the anticalculus agent is a polyphosphate. A polyphosphate is generally understood to consist of two or more phosphate molecules arranged primarily in a linear configuration, although some cyclic derivatives may be present. Linear polyphosphates correspond to (X PO₃) ₙ where n is about 2 to about 125, wherein preferably n is greater than 4, and X is for example sodium, potassium, etc. For (X PO₃) ₙ when n is at least 3 the polyphosphates are glassy in character. Counterions for these phosphates may be the alkali metal, alkaline earth metal, ammonium, C₂-C₆ alkanolammonium and salt mixtures. Polyphosphates are generally employed as their wholly or partially neutralized water soluble alkali metal salts such as potassium, sodium, ammonium salts, and mixtures thereof. The inorganic polyphosphate salts include alkali metal (e.g. sodium) tripolyphosphate, tetrapolyphosphate, dialkyl metal (e.g. disodium) diacid, trialkyl metal (e.g. trisodium) monoacid, potassium hydrogen phosphate, sodium hydrogen phosphate, and alkali metal (e.g. sodium) hexametaphosphate, and mixtures thereof. Polyphosphates larger than tetrapolyphosphate usually occur as amorphous glassy materials. In one embodiment the polyphosphates are those manufactured by FMC Corporation which are commercially known as Sodaphos (n≈6), Hexaphos (n≈13), and Glass H (n≈21), and mixtures thereof. The present compositions will typically comprise from about 0.5% to about 20%, in one embodiment from about 4% to about 15%, in yet another embodiment from about 6% to about 12%, by weight of the composition of polyphosphate.

The phosphate sources are described in more detail in Kirk & Othmer, Encyclopedia of Chemical Technology, Fourth Edition, Volume 18, Wiley-Interscience Publishers (1996), pages 685-707, incorporated herein by reference in its entirety, including all references incorporated into Kirk & Othmer.

In one embodiment the polyphosphates are the linear "glassy" polyposphates having the formula:

XO(XPO₃)ₙX

wherein X is sodium or potassium; and n averages from about 6 to about 125.

In one embodiment, when n is at least 2 in either of the above polyphosphate formulas, the level of anticalculus agent is from about 4.5% to about 40%, in another embodiment is from about 5% to about 25%, and in even another embodiment is from about 8% to about 15%, by weight of the composition. Polyphosphates are further disclosed in U.S. 4,913,895.

### Pyrophosphate

The pyrophosphate salts useful in the present compositions include, alkali metal pyrophosphates, di-, tri-, and mono-potassium or sodium pyrophosphates, dialkali metal pyrophosphate salts, tetraalkali metal pyrophosphate salts, and mixtures thereof. In one embodiment the pyrophosphate salt is selected from the group consisting of trisodium pyrophosphate, disodium dihydrogen pyrophosphate (Na₂H₂P₂O₇), dipotassium pyrophosphate, tetrasodium pyrophosphate (Na₄P₂O₇), tetrapotassium pyrophosphate (K₄P₂O₇), and mixtures thereof. The pyrophosphate salts described in U.S. Patent 4,515,772, issued May 7, 1985, and U.S. Pat. No. 4,885,155, issued December 5, 1989, both to Parran et al.. The pyrophosphate salts are described in more detail in Kirk & Othmer, Encyclopedia of Chemical Technology, Third Edition, Volume 17, Wiley-Interscience Publishers (1982), pages 685-707.

In one embodiment, the compositions of the present invention comprise tetrasodium pyrophosphate. Tetrasodium pyrophosphate may be the anhydrous salt form or the decahydrate form, or any other species stable in solid form in the present compositions. The salt is in its solid particle form, which may be its crystalline and/or amorphous state, with the particle size of the salt preferably being small enough to be aesthetically acceptable and readily soluble during use.

The level of pyrophosphate salt in the compositions of the present invention is any safe and effective amount, and is generally from about 1.5% to about 15%, in another embodiment from about 2% to about 10%, and yet in another embodiment from about 3% to about 8%, by weight of the composition.

Optional agents to be used in place of or in combination with the pyrophosphate salt include such known materials as synthetic anionic polymers, including polyacrylates and copolymers of maleic anhydride or acid and methyl vinyl ether (e.g., Gantrez), as described, for example, in U.S. Patent 4,627,977, to Gaffar et al., as well as, e.g., polyamino propoane sulfonic acid (AMPS), zinc citrate trihydrate, polyphosphates (e.g., tripolyphosphate; hexametaphosphate), diphosphonates (e.g., EHDP; AHP), polypeptides (such as polyaspartic and polyglutamic acids), and mixtures thereof.

### Antimicrobial Agents

Antimicrobial antiplaque agents may also by optionally present in the present compositions. Such agents may include, but are not limited to, triclosan, 5-chloro-2-(2,4-dichlorophenoxy)-phenol, as described in The Merck Index, 11th ed. (1989), pp. 1529 (entry no. 9573) in U.S. Patent No. 3,506,720, and in European Patent Application No. 0,251,591 of Beecham Group, PLC, published January 7, 1988; chlorhexidine (Merck Index, no. 2090), alexidine (Merck Index, no. 222; hexetidine (Merck Index, no. 4624); sanguinarine (Merck Index, no. 8320); benzalkonium chloride (Merck Index, no. 1066); salicylanilide (Merck Index, no. 8299); domiphen bromide (Merck Index, no. 3411); cetylpyridinium chloride (CPC) (Merck Index, no. 2024; tetradecylpyridinium chloride (TPC); N-tetradecyl-4-ethylpyridinium chloride (TDEPC); octenidine; delmopinol, octapinol, and other piperidino derivatives; effective antimicrobial amounts of essential oils and combinations thereof for example citral, geranial, and combinations of menthol, eucalyptol, thymol and methyl salicylate; antimicrobial metals and salts thereof for example those providing zinc ions, stannous ions, copper ions, and/or mixtures thereof; bisbiguanides, or phenolics; antibiotics such as augmentin, amoxicillin, tetracycline, doxycycline, minocycline, and metronidazole; and analogs and salts of the above antimicrobial antiplaque agents; anti-fungals such as those for the treatment of *candida albicans.* If present, these agents generally are present in a safe and effective amount for example from about 0.1% to about 5% by weight of the compositions of the present invention.

### Anti-inflammatory Agents

Anti-inflammatory agents may also be present in the oral compositions of the present invention. Such agents may include, but are not limited to, non-steroidal anti-inflammatory agents such as aspirin, ketorolac, flurbiprofen, ibuprofen, naproxen, indomethacin, aspirin, ketoprofen, piroxicam and meclofenamic acid, COX-2 inhibitors such as valdecoxib, celecoxib and rofecoxib, and mixtures thereof. If present, the anti-inflammatory agents generally comprise from about 0.001% to about 5% by weight of the compositions of the present invention. Ketorolac is described in U.S. Patent 5,626,838, issued May 6, 1997.

### H-2 Antagonists

The present invention may also comprise a safe and effective amount of a selective H-2 antagonist including compounds disclosed in U.S. Patent 5,294,433, Singer et al., issued March 15, 1994.

### Surfactants

The present composition may also optionally contain suitable surfactants. Surfactants may provide better dispersion of the present composition in the oral cavity. Surfactants include nonionic, anionic, amphoteric, cationic, zwitterionic, synthetic detergents, and mixtures thereof. Many suitable nonionic and amphoteric surfactants are disclosed by U.S. Pat. Nos. 3,988,433 to Benedict; U.S. Patent 4,051,234, issued September 27, 1977, and many suitable nonionic surfactants are disclosed by Agricola et al., U.S. Patent 3,959,458, issued May 25, 1976.

The present composition typically comprises a safe and effective amount of a surfactant, in another embodiment comprises from about 0.001% to about 20%, in another embodiment from about 0.05% to about 9%, and in even another embodiment from about 0.1% to about 5% by weight of the composition.

### Sweetening Agents, Coolants, Warming Agents, and Salivating Agents

Sweetening agents which can optionally be used include sucralose, sucrose, glucose, saccharin, dextrose, levulose, lactose, mannitol, sorbitol, fructose, maltose, xylitol, saccharin salts, thaumatin, aspartame, D-tryptophan, dihydrochalcones, acesulfame and cyclamate salts, especially sodium cyclamate and sodium saccharin, and mixtures thereof. A composition preferably contains from about 0.1% to about 10% of these agents, in another embodiment from about 0.1% to about 1%, and in yet another embodiment from about 0.5 to about 2%, by weight of the composition.

In addition to flavoring and sweetening agents, coolants, salivating agents, warming agents, and numbing agents can be used as optional ingredients in compositions of the present invention. These agents are present in the compositions at a level of from about 0.001% to about 10%, in another embodiment from about 0.1% to about 3%, and in yet another embodiment from about 0.5 to about 2%, by weight of the composition.

The coolant can be any of a wide variety of materials. Included among such materials are carboxamides, menthol, ketals, diols, and mixtures thereof. Preferred coolants in the present compositions are the paramenthan carboxyamide agents such as N-ethyl-p-menthan-3-carboxamide, known commercially as "WS-3", N,2,3-trimethyl-2-isopropylbutanamide, known as "WS-23," and mixtures thereof. Additional preferred coolants are selected from the group consisting of menthol, 3-1-menthoxypropane-1,2-diol known as TK-10 manufactured by Takasago, menthone glycerol acetal known as MGA manufactured by Haarmann and Reimer, and menthyl lactate known as Frescolat® manufactured by Haarmann and Reimer. The terms menthol and menthyl as used herein include dextro- and levorotatory isomers of these compounds and racemic mixtures thereof. TK-10 is described in U.S. Pat. No. 4,459,425, Amano et al., issued 7/10/84. WS-3 and other agents are described in U.S. Pat. No. 4,136,163, Watson, et al., issued Jan. 23, 1979.

Salivating agents of the present invention include Jambu® manufactured by Takasago. Warming agents include capsicum and nicotinate esters, such as benzyl nicotinate. Numbing agents include benzocaine, lidocaine, clove bud oil, ethanol, and mixtures thereof.

### Sensitivity Agents/Anesthetic Agents

Anti-pain or desensitizing agents can also be present in a safe and effective amount in the compositions of the present invention. Analgesics are agents that relieve pain by acting centrally to elevate pain threshold without disturbing consciousness or altering other sensory modalities. Such agents may include, but are not limited to, strontium chloride, potassium nitrate, sodium nitrate, sodium fluoride, acetanilide, phenacetin, acertophan, thiorphan, spiradoline, aspirin, codeine, thebaine, levorphenol, hydromorphone, oxymorphone, phenazocine, fentanyl, buprenorphine, butaphanol, nalbuphine, pentazocine, natural herbs such as gall nut, Asarum, Cubebin, Galanga, scutellaria, Liangmianzhen, Baizhi, etc. Anesthetic agents, or topical analgesics, such as acetaminophen, sodium salicylate, trolamine salicylate, lidocaine and benzocaine may also be present. These analgesic actives are described in detail in Kirk-Othmer, Encyclopedia of Chemical Technology, Fourth Edition, Volume 2, Wiley-Interscience Publishers (1992), pp. 729-737.

### Composition Use

The present invention relates to improved diagnostic methods and compositions for the detection of demineralized lesions. The present invention offers the following advantages. First, the present invention offers a safer and less expensive alternative to some of the diagnostic procedures currently available. It requires only visual observation with visible light and does not require (though they can optionally be used) the use of lasers, other types of exaggerated light sources, or X-rays to highlight the demineralization lesions. Also, the present invention is convenient to use and may be used either at home by the consumer or in the dental office by dental professionals. It can also serve as a means for detecting demineralization lesions during clinical testing of various caries (prevention or treatment) products. Further, the present invention can selectively detect frank caries lesions to aid the dental professional in deciding how much of the tooth surface to remove, preventing the removal of sound portions of the tooth during restoration. Furthermore, the present invention provides detection of early caries lesions to enable early treatment with remineralization agents at a point where some reversal or remineralization of the condition may be possible. If the degree of demineralization is not too extensive, e.g. is reversible, then treatment of the lesion may prevent the formation of a frank caries lesion.

Importantly, the present invention can be used as a means to more frequently monitor the progress of remineralization treatment, such as the use of any oral care product containing fluoride ions. It can monitor how much of the tooth is being remineralized after the application of remineralization agents or improved oral hygiene practices by the subject (e.g. better brushing, flossing, etc.).

Lastly, the present invention provides an incentive for subjects to adhere to good oral hygiene practices. This invention provides a "consumer friendly" way of monitoring progress and showing improvement and success with a remineralization treatment regimen. This may further motivate the subject to continue therapy and remain compliant with treatment. Better compliance may avoid the progression to irreversible lesions and avoid drilling, removal of tooth structure, and filling the lesion (e.g. restoration).

The compositions and methods of the present invention may be applied to the oral cavity in any desired form, including but not limited to, gel (either brushed on teeth or applied with applicator or fingers), powder, spray, mousse, aerosol, compositions used with an integral carrier, (e.g. a non-dissolvable backing strip), tablet, chewable tablets, capsules, chewable forms with liquid filled centers, quick dissolving discs or tablets, chewing gums, edible films, mouthrinses, solutions, and the like where the product is applied to the tooth surface. Rinses, solutions, gels may be applied to one or more tooth surfaces with a brush, sponge, swab, pipette, fingers, or any other device that can deliver a uniform dose of the composition.

The first composition and the second composition are applied to one or more teeth sequentially.

With sequential administration, the subject applies or administers the first dye composition to one or more teeth, or to the oral cavity. If the exposure is too long, selective staining may not be achieved. Then the subject allows the first composition to contact the oral surfaces, especially the teeth, for at least 5 seconds to about 1 minute, in another embodiment from about 6 seconds to about 30 seconds, and in yet another embodiment from about 10 seconds to about 20 seconds. Thereafter the subject spits the composition out of the oral cavity and optionally rinses the oral cavity with water (swishes for 30 seconds to 1 minute, then expectorates). The subject then applies the second dye composition to the oral cavity and allows the composition to contact the oral surfaces, especially the teeth, for at least 5 seconds to about 1 minute, in another embodiment from about 6 seconds to about 30 seconds, and in yet another embodiment from about 10 seconds to about 20 seconds. The subject then spits the composition out of the oral cavity. The subject then rinses the oral cavity with water for about 15 seconds to about 1 minute, then expectorates. Thereafter the subject or dental professional visually observes the teeth and observes the degree of demineralized lesions or the mineralization health of the teeth. This procedure can be repeated as needed by the subject or dentist to monitor treatment effectiveness. Generally the frequency of use is from about every day to about once every 12 months, in another embodiment, about every week to about every 6 months. After observation, the dye is generally removed from the teeth or other oral soft tissues through brushing or rinsing, e.g. with water.

For each tooth generally the subject uses a volume of each of the first phase and the second phase of from about 0.05 mil to about 5 mils, in another embodiment from about 0.1 mils to about 1 mils, in another embodiment from about 0.15 mils to about 0.3 mils.

In one embodiment, prior to applying the first dye composition the subject removes plaque or calculus from the teeth, via methods or agents described herein.

Edible film compositions and methods of making them are disclosed in U.S. 6,419,903, Xu et al., issued July 16,2002, Colgate Palmolive Company; U.S. Patent Application No. 2003/0053962, published March 20, 2003, Zerbe et al., LTS Lohmann; WO 02/02085 A2, LTS Lohmann Therapie-Systeme AG, published Jan. 10, 2002; U.S. Patent Application No. 2001/022964, published Sept. 20,2001, Leung et al., Warner-Lambert Company; U.S. 2002/0131990, published Sept. 19, 2002, Wm. Wrigley Jr. Company; US 2003/0054039, Zyck et al., published March 20, 2003, Wm. Wrigley Jr. Company; and US 2002/0127254 (Lavipharm); U.S. 5,948,430, U.S. 6,284,264B1, U.S. 6,177,096B1, LTS Lohmann.

Confectionery and chewing gum compositions and methods of making them are disclosed in U.S. 5,702,687, Church & Dwight; U.S. 4,170,632, General Mills; U.S. 4,151,270, Wm. Wrigley Jr.; U.S. 5,306,519 Universal Foods; U.S. 5,194,288 Universal Foods; U.S. 5,958,472 Warner Lambert; U.S. 4,931,295 Wm. Wrigley, Jr.; U.S. 5,017,385 P&G; U.S. 5,158,789 ICA Americas; U.S. 4,976,972 Wm Wrigley Jr.; EP 453,402 Warner Lambert; U.S. 4,997,654 Warner Lambert; U.S. 4,513,012 Warner Lambert; U.S. 4,513,012 Warner Lambert; U.S. Application No. 2003/00728 41-A1, published April 17, 2003, P&G.

In one embodiment the dosage form is selected from the group consisting of gels, solutions, rinses, gels combined with an integral carrier (e.g. non dissolvable backing strip), forms that provide direct application to one or more teeth while minimizing dye composition exposure to the soft tissue of the oral mucosa, and mixtures thereof. These dosage forms may be applied directly to 1 or more teeth.

Gels on a non-dissolvable backing strips and methods of making them are disclosed above.

The compositions of this invention are useful for both human and animal (e.g. pets, zoo, or domestic animals) applications.

### EXAMPLES

The following non-limiting examples further describe embodiments within the scope of the present invention.

### EXAMPLE I (Solutions)

| Ingredient | Example 1 | | Example 2 | | Example 3 | |
|---|---|---|---|---|---|---|
| | First Composition (% by wt.) | Second Composition (% by wt.) | First Composition (% by wt.) | Second Composition (% by wt.) | First Composition (% by wt.) | Second Composition (% by wt) |
| Blue Dye¹ | 0.5 | | 0.1 | | 1.0 | |
| Red Dye² | | 0.5 | | | | 1.0 |
| Yellow Dye³ | | | | 0.4 | | |
| Proplyene Glycol | 99.45 | 99.45 | 99.87 | 99.57 | 98.95 | 98.95 |
| Flavor⁴ | 0.05 | 0.05 | 0.03 | 0.03 | 0.05 | 0.05 |

| | | | | | | |
|---|---|---|---|---|---|---|
| ¹Acid Blue 9, Food Blue 1, or Food Blue 2. ²Acid Red 52 ³Yellow Dye 5 ⁴Flavor is selected from spearmint, eugenol, peppermint and/or cinnamon. | | | | | | |

### EXAMPLE II (Gels)

| | | | | | | |
|---|---|---|---|---|---|---|
| Ingredient | Example 4 | | Example 5 | | Example | 6 |
| | First Composition (% by wt.) | Second Composition (% by wt.) | First Composition (% by wt.) | Second Composition (% by wt.) | First Composition (% by wt) | Second Composition (% by wt.) |
| Blue Dye¹ | 0.5 | | 0.1 | | 1.0 | |
| Red Dye² | | 0.5 | | | | 1.0 |
| Yellow Dye³ | | | | 0.4 | | |
| Proplyene Glycol | 97.45 | 97.45 | 98.87 | 98.57 | 96.45 | 96.45 |
| Flavor⁴ | 0.05 | 0.05 | 0.03 | 0.03 | 0.05 | 0.05 |
| Carbopol 956⁵ | 2.0 | 2.0 | | | 2.5 | 2.5 |
| HPMC⁶ | | | 1.0 | 1.0 | | |

| | | | | | | |
|---|---|---|---|---|---|---|
| ¹Acid Blue 9, Food Blue 1 or Food Blue 2. ²Acid Red 52 ³Yellow Dye 5. ⁴Flavor is selected from spearmint, eugenol, peppermint and/or cinnamon. ⁶Available from BF Goodrich. | | | | | | |

In the above Examples the first and second compositions are used in equal parts. The above solutions and gels are painted on all of the teeth with an applicator brush, disposable pipette, sponge, swab, or other device that can deliver a uniform dose of the composition.

### Sequential Administration of Dyes:

A human subject dispenses about 0.1 - 02 mils of the first compositions on each tooth to be analyzed and leaves it on for about 10 - 20 seconds. Then the subject expectorates any excess solution or gel from the oral cavity and optionally rinses the oral cavity for about 45 seconds with water. The subject thereafter dispenses 0.1 to 0.2 mils of the second phase on each tooth to be analyzed and waits for about 10 - 20 seconds. Then the subject expectorates the excess solution or gel from the oral cavity and rinses the oral cavity for about 45 seconds with water. Finally the subject visually observes all tooth surfaces to assess the degree of demineralized lesion of the tooth surfaces that are stained by the dye. Then after brushing the teeth twice a day with a fluoride toothpaste such as Crest® Cavity protection, having fluoride ions present, the subject repeats the method every 6 months. The method provides a means to observe the number of demineralized lesions of the pretreatment teeth versus post treatment.

### Concurrent Administration of Dyes: (not encompassed by the scope of the claims)

Alternatively, a human subject dispenses about 0.1- 0.2 mils of the first on each tooth to be analyzed. The subject leaves the first composition on the teeth for about 10 - 20 seconds, and then dispenses about 0.1- 0.2 mils of the second composition on the same teeth treated with the first composition. The subject leaves the second composition on the teeth for about 10 to about 20 seconds and thereafter rinses the oral cavity for about 45 seconds with water.
Then the subject visually observes all tooth surfaces to assess the degree of demineralized lesion of the tooth surfaces that are stained by the dye. Then the subject brushes the teeth twice a day with a fluoride toothpaste such as Crest® Cavity protection, having fluoride ions present. The subject repeats the method every 6 months. The number of demineralized lesions is reduced. The method provides a means to observe the reduced number of demineralized lesions of the pretreatment teeth versus post treatment.

## Claims

1. The use of a blue dye, together with a red dye or a yellow dye, in the manufacture of a kit for visually highlighting demineralization lesions in tooth surfaces wherein the kit comprises:
a) a first composition comprising from 0.001% to 10%, by weight of the composition of a blue dye or mixtures thereof; and
b) a second composition comprising from 0.001% to 10%, by weight of the composition of a red dye or a yellow dye, or mixtures thereof;
wherein the first and second compositions each comprise a safe and effective amount of a solvent for the dyes, wherein the dyes are soluble in the solvent and the visual highlighting comprises sequentially applying the first composition, followed by the second composition, to the tooth surface.

2. The use of Claim 1 wherein the first and second compositions comprise a flavour.

3. The use of Claim 1 or Claim 2 wherein the blue dye is selected from Acid Blue 9, Food Blue 1, Food Blue 2 and mixtures thereof.

4. The use of any of Claims 1 to 3 wherein the red or yellow dye is C.I. Acid Red 52.

5. The use of any of Claims 1 to 4 wherein the solvent is selected from propylene glycol, 1,3-propylene glycol, 1,2-propylene glycol, dipropylene glycol and mixtures thereof.

6. The use of any of Claims 1 to 5 wherein the compositions additionally comprise a safe and effective amount of a pellicle disruption agent, an oral care active agent, a buffer system, a surfactant, or mixtures thereof.

7. The use of any of Claims 1 to 6 wherein at least one of the compositions is releasably associated with an integral carrier which is a water insoluble strip of material.

8. The use of Claim 7 wherein the at least one composition is in the form of a gel.

## Patentansprüche

1. Verwendung eines blauen Farbstoffs zusammen mit einem roten Farbstoff oder einem gelben Farbstoff bei der Herstellung eines Kits für die visuelle Betonung von Demineralisierungsläsionen in Zahnoberflächen, wobei das Kit umfasst:
a) eine erste Zusammensetzung, die von 0,001 % bis 10 %, bezogen auf das Gewicht der Zusammensetzung, einen blauen Farbstoff oder Mischungen davon umfasst; und
b) eine zweite Zusammensetzung, die von 0,001 % bis 10 %, bezogen auf das Gewicht der Zusammensetzung, einen roten Farbstoff oder einen gelben Farbstoff oder Mischungen davon umfasst;
wobei die ersten und zweiten Zusammensetzungen jeweils eine sichere und wirksame Menge eines Lösungsmittels für die Farbstoffe umfassen, wobei die Farbstoffe in dem Lösungsmittel löslich sind, und die visuelle Betonung die sequentielle Auftragung der ersten Zusammensetzung, gefolgt von der zweiten Zusammensetzung, auf die Zahnoberfläche umfasst.

2. Verwendung nach Anspruch 1, wobei die ersten und zweiten Zusammensetzungen einen Geschmacksstoff umfassen.

3. Verwendung nach Anspruch 1 oder Anspruch 2, wobei der blaue Farbstoff ausgewählt ist aus Acid Blue 9, Food Blue 1, Food Blue 2 und deren Mischungen.

4. Verwendung nach einem der Ansprüche 1 bis 3, wobei der rote oder gelbe Farbstoff C.I. Acid Red 52 ist.

5. Verwendung nach einem der Ansprüche 1 bis 4, wobei das Lösungsmittel ausgewählt ist aus Propylenglycol, 1,3-Propylenglycol, 1,2-Propylenglycol, Dipropylenglycol und deren Mischungen.

6. Verwendung nach einem der Ansprüche 1 bis 5, wobei die Zusammensetzungen zusätzlich eine sichere und wirksame Menge eines Biofilm-Sprengmittels, eines Mundpflegewirkstoffs, eines Puffersystems, eines Tensids oder von Mischungen davon umfasst.

7. Verwendung nach einem der Ansprüche 1 bis 6, wobei mindestens eine der Zusammensetzungen lösbar mit einem integralen Träger assoziiert ist, der ein Streifen aus wasserunlöslichem Material ist.

8. Verwendung nach Anspruch 7, wobei die mindestens eine Zusammensetzung in Form eines Gels vorliegt.

## Revendications

1. Utilisation d'une teinture bleue, conjointement avec une teinture rouge ou une teinture jaune, dans la fabrication d'une trousse pour mettre visuellement en évidence des lésions de déminéralisation sur des surfaces dentaires dans laquelle la trousse comprend :
a) une première composition comprenant de 0,001 % à 10 % en poids de la composition d'une teinture bleue ou de ses mélanges ; et
b) une deuxième composition comprenant de 0,001 % à 10 % en poids de la composition d'une teinture rouge ou d'une teinture jaune, ou leurs mélanges ;
dans laquelle les première et deuxième compositions comprennent chacune une quantité sûre et efficace d'un solvant pour les teintures, dans laquelle les teintures sont solubles dans le solvant et la mise en évidence visuelle comprend une application séquentielle de la première composition, suivie par la deuxième composition, sur la surface dentaire.

2. Utilisation selon la revendication 1, dans laquelle les première et deuxième compositions comprennent un arôme.

3. Utilisation selon la revendication 1 ou la revendication 2, dans laquelle la teinture bleue est choisie parmi Acid Blue 9, Food Blue 1, Food Blue 2 et leurs mélanges.

4. Utilisation selon l'une quelconque des revendications 1 à 3, dans laquelle la teinture rouge ou jaune est C.I. Acid Red 52.

5. Utilisation selon l'une quelconque des revendications 1 à 4, dans laquelle le solvant est choisi parmi le propylène glycol, le 1,3-propylène glycol, le 1,2-propylène glycol, le dipropylène glycol et leurs mélanges.

6. Utilisation selon l'une quelconque des revendications 1 à 5, dans laquelle les compositions comprennent, en outre, une quantité sûre et efficace d'un agent de dislocation de pellicule, un agent actif de soins bucco-dentaires, un système de tampon, un agent tensioactif, ou leurs mélanges.

7. Utilisation selon l'une quelconque des revendications 1 à 6, dans laquelle au moins une des compositions est associée de manière libérable à un support d'un seul tenant qui est une bande de matériau insoluble dans l'eau.

8. Utilisation selon la revendication 7, dans laquelle la composition au moins unique est sous la forme d'un gel.
